# DEMANDE DE BREVET EUROPEEN

(11) **EP 0 725 274 A1**
(43) Date de publication de la demande: **07.08.1996**
(21) Numéro de dépôt: 96400213.3
(22) Date de dépôt: 31.01.1996
(51) Int. Cl.: G01N 33/487, A61M 1/36

(54) **Dispositif de mesure de fluides corporels installé sur une installation extra corporelle**

(30) Priorité: 02.02.1995 FR 9501222
(71) Demandeur: LABORATOIRE EUGEDIA, F-60230 Chambly (FR)
(72) Inventeur: Jussiaux, Philippe, F-60230 Chambly (FR)

(57) **Abrégé**

La présente invention propose un dispositif de mesure et/ou de contrôle (24) placé sur un circuit extra corporel de fluides corporels en particulier sur le circuit de circulation sanguine externe d'une machine d'hémodialyse.

Grâce au système de stérilisation de l'invention, le dispositif peut rester installé sur la machine, la stérilisation étant effectuée *in situ*. L'invention prévoit l'installation de moyens de confinement (15) et de stérilisation (16, 17, 18, 20). On présente en particulier un système de stérilisation *in situ* par la chaleur sèche.

## Description

La présente invention a pour objet un dispositif de mesure des fluides corporels, du sang en particulier, installé sur une installation de circulation extra corporelle telle qu'une machine d'hémodialyse.

Les installations d'hémodialyse habituelles sont constituées d'un bâti sur lequel sont fixés tous les dispositifs nécessaires à la purification du sang du patient. Ceux-ci sont à usage unique et ils sont jetés après utilisation pour éviter tout risque de contamination d'un patient par le patient précédent. Les dispositifs concernés sont le dialyseur (c'est à dire le rein artificiel proprement dit) et, essentiellement, des lignes, c'est à dire des tubulures, généralement en matière plastique, tous les autres éléments comme les pompes, les manomètres etc. sont soit exclus du contact avec le sang du patient, soit également jetables.

Cependant, il serait très intéressant, sur une installation d'hémodialyse, comme sur toute installation prévue pour réaliser la circulation extra corporelle de fluides corporels, de pouvoir effectuer des mesures ou simplement, de pouvoir intervenir dans le circuit extra-corporel avec des appareils, des dispositifs, des cellules de mesure sans devoir les jeter après chaque usage.

L'invention se donne pour tâche de permettre sur une installation où circulent des fluides corporels, l'utilisation, dans le circuit extra-corporel, de dispositifs non jetables et, donc réutilisables, sans risque de contamination d'un patient par un autre.

Pendant les séances d'hémodialyse, on souhaite pouvoir effectuer de multiples mesures et contrôles qui permettent, d'une part de gérer la dialyse en agissant sur les différents paramètres de manière à ce que l'opération se déroule conformément au programme décidé par le praticien et d'autre part, d'éviter tout malaise ou désagrément pour le patient. Il existe de nombreuses possibilités pour réaliser de tels contrôles selon les méthodes habituelles, la seule difficulté réside dans le fait que, pour éviter les contaminations du sang du malade, il est indispensable de n'utiliser que des dispositifs stériles jetables car une opération de démontage, de stérilisation puis de remontage de l'instrument de mesure ou de contrôle entre le traitement d'un patient et celui du suivant serait trop compliquée et reviendrait trop cher. Cette servitude limite le nombre de contrôles qu'il est possible de réaliser et entraîne une dépense élevée pour ceux qu'on effectue réellement. C'est un but de l'invention que de permettre des mesures plus faciles même avec des appareils compliqués.

Les méthodes de stérilisation connues sont nombreuses. Certaines, classiques, utilisent la chaleur seule ou la chaleur humide, de préférence sous pression, et d'autres, les rayonnements, X ou γ, ultraviolets ou même, corpusculaires (β). Le document EP-A-0 313 409 propose ainsi une méthode de stérilisation utilisant le rayonnement ultraviolet émis par une source à base d'halogénure de gallium et de mercure en présence d'un oxyde métallique. De même, le document JP-A-62-204 754 prévoit d'isoler, par exemple dans un sac, les appareils à stériliser puis de les irradier avec des rayonnements radioactifs en présence d'un agent désoxygénant tel que de l'oxyde de fer activé.

Un contrôle d'un fluide circulant à l'extérieur du corps qui présente un intérêt très grand est celui de l'hématocrite, c'est à dire de la concentration du sang en globules rouges, en particulier pendant les séances d'hémodialyse, en effet c'est le contrôle de ce paramètre qui permet d'extraire puis de réintroduire dans l'organisme du patient, la quantité de liquide voulue. Mais la mesure de l'hématocrite présente un intérêt supplémentaire : elle permet la prévention des incidents graves pendant la dialyse tels que les collapsus cardio-vasculaires. Différentes méthodes pour accéder à cette grandeur, l'hématocrite, ont été proposées, mais la plupart réalisent les mesures sur le patient lui-même. Le document EP-A-0 551 043 en revanche, propose d'effectuer directement la mesure de l'hématocrite, soit optiquement, soit électriquement, sur le circuit sanguin extra-corporel. La méthode préférée dans ce document est celle de la mesure de l'impédance, elle nécessite qu'on introduise sur une ligne stérile jetable deux aiguilles qui jouent le rôle d'électrodes. Cette méthode, outre son coût, présente plusieurs inconvénients, l'opération de montage est délicate, la précision dans le positionnement des aiguilles (les électrodes) dont l'écart intervient dans la mesure, n'est pas toujours assurée etc. L'invention se donne également pour tâche d'améliorer les conditions de cette mesure de l'hématocrite sur le circuit sanguin extra-corporel pendant l'hémodialyse.

Pour résoudre les différents problèmes posés, l'invention propose un dispositif de mesure et/ou de contrôle placé sur un circuit extra-corporel de fluides corporels qui comporte des moyens de confinement et de stérilisation. Selon une variante, le moyen de stérilisation est la chaleur et le confinement est réalisé dans une enceinte.

L'invention prévoit également dans l'une de ses variantes que la source de chaleur soit une résistance chauffante placée de préférence en partie basse. Une autre variante de l'invention prévoit que la chaleur soit celle d'un courant d'air chaud traversant l'enceinte. Dans le cas de l'utilisation de la chaleur comme moyen de stérilisation, de préférence le confinement est réalisé par des courants de convection dans l'enceinte. Dans tous les cas d'utilisation de chaleur comme agent de stérilisation, il est prévu qu'un détecteur de température soit placé à proximité du dispositif et qu'on l'utilise comme moyen de contrôle de la stérilisation.

L'utilisation de la chaleur est traditionnelle comme agent de stérilisation et son utilisation sur une installation de dialyse ne pose pas de problèmes nouveaux. Le contrôle de l'efficacité de la stérilisation est également très simple dans le cas de l'utilisation de la chaleur car il suffit de vérifier qu'une température suffisante a été atteinte pendant suffisamment longtemps.

L'invention propose également une variante dans laquelle le confinement est réalisé dans un récipient étanche et où le moyen de stérilisation est un liquide désinfectant et une autre où l'agent de stérilisation est un rayonnement et où le confinement est obtenu par un rayonnement pluridirectionnel.

L'invention s'applique spécialement à une cellule de mesure installée sur une machine d'hémodialyse, notamment pour la mesure de l'impédance du sang qui comporte un canal où circule le sang. Alors, en cas d'utilisation de chaleur, le canal où circule le sang est placé de préférence sensiblement verticalement.

Les figures et la description qui suivent permettront de comprendre l'invention et d'en saisir tous les avantages.

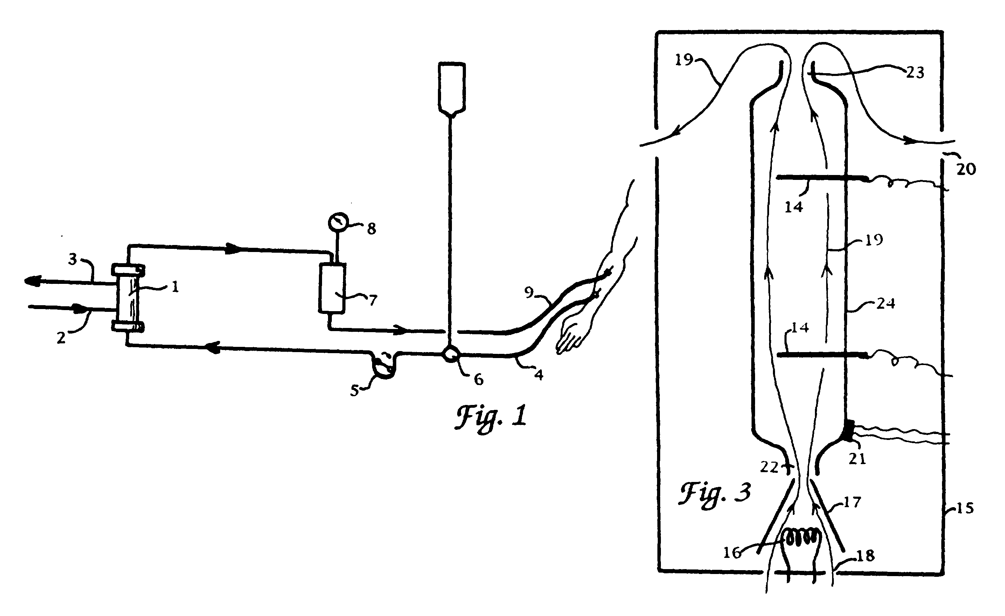

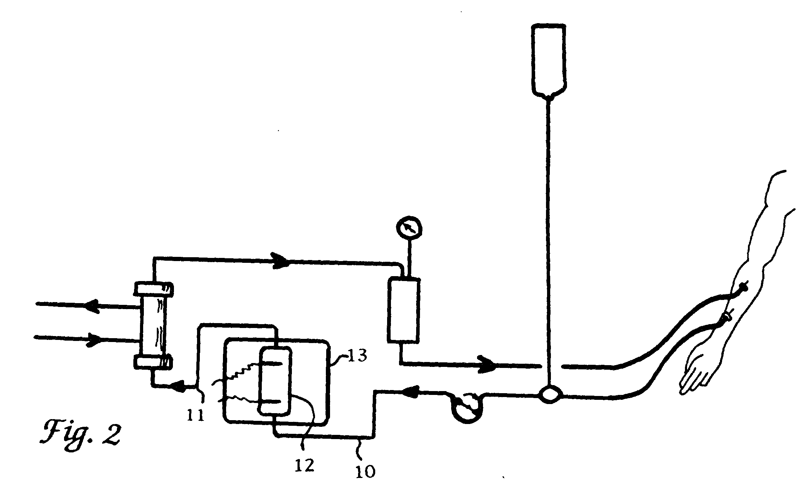

**La figure 1** montre le schéma d'une installation d'hémodialyse traditionnelle, **la figure 2**, la même installation avec, en plus, le dispositif de l'invention et, **la figure 3**, un mode de réalisation du dispositif, celui qui utilise la chaleur comme moyen de stérilisation.

L'installation de dialyse de **la figure 1** comporte en 1 le rein artificiel proprement dit, où se fait l'échange entre le sang du patient et le dialysat, celui-ci est introduit en 2 et évacué en 3. Le sang quitte le patient par la ligne artérielle 4 et, avant d'être entraîné par la pompe 5, reçoit éventuellement un apport de liquide physiologique en 6. Le sang traverse alors le dialyseur 1 où il est purifié. Il transite enfin par un piège à bulles 7 équipé d'un manomètre 8 avant d'être réintroduit dans le corps par la ligne veineuse 9. Pour éviter tout risque de contamination d'un patient par un autre, tous les dispositifs de la figure, même le dialyseur 1 dans la plupart des cas, sont stériles et à usage unique.

Lorsqu'on utilise des techniques de mesure et/ou de contrôle il faut absolument prendre des précautions particulières. Ou bien, il s'agit d'une intervention exceptionnelle et alors, on procède à une stérilisation très soignée de tous les appareils avant leur installation et, en plus, comme c'est le cas avec les dialyseurs réutilisables, ils sont réservés à un seul et même patient. Ou bien s'il s'agit d'une mesure de routine, tout élément du dispositif de mesure qui est en contact avec le sang du patient est stérile et à usage unique. C'est ainsi que pour mettre en oeuvre la méthode de la demande de brevet EP-A-0 551 043, dans le cas de l'utilisation d'une mesure d'impédance électrique, un élément de ligne jetable est utilisé comme cellule de mesure. On y introduit deux aiguilles hypodermiques qui servent d'électrodes sur lesquelles viennent se raccorder les conducteurs électriques pour l'alimentation et la mesure. L'opération est délicate, le positionnement des électrodes, très important pour le résultat de la mesure, difficile à réaliser et le matériel à jeter, cher.

**La figure 2** montre une installation d'hémodialyse identique à celle de la figure 1, mais où on a inséré un dispositif conforme à l'invention. Il s'agit ici d'une cellule de mesure le l'impédance du sang avec stérilisation par la chaleur. Sur la figure, le dispositif est représenté branché, en fonctionnement, raccordé entre deux lignes, 10 en amont et 11 en aval. Ces lignes sont stériles et jetables, elles sont raccordées à la cellule de mesure 12. Cette cellule est montée à poste fixe, verticalement, sur la machine d'hémodialyse. Sur la figure, on a représenté en 13 les limites de l'enceinte de confinement. Le confinement est ici, dans l'acception la plus générale du terme, l'opération qui consiste à concentrer les moyens de stérilisation sur le dispositif à traiter. Dans le cas d'utilisation de la chaleur et dans celui de fluides stérilisants (liquides ou gaz), l'opération consiste à isoler le dispositif du reste de l'appareil pour justement pouvoir concentrer la chaleur ou le fluide sur le dispositif à traiter. Dans le cas d'utilisation de rayonnements stérilisants, comme les rayons UV, X ou γ, le confinement consiste à obtenir un rayonnement qui irradie toutes les surfaces à traiter. Le confinement nécessite alors de disposer plusieurs sources ou d'utiliser des dispositifs optiques divers tels que des concentrateurs, des réflecteurs etc. pour permettre une irradiation pluridirectionnelle qui permette aux rayonnements d'agir partout.

Sur **la figure 3** on a représenté une cellule de mesure de l'impédance conformément au document EP-A-0 551 043 dont l'enseignement est incorporé au présent texte. On voit en 13 la cellule proprement dite constituée d'un tube cylindrique en verre ou par exemple en plastique résistant aux agents et aux techniques de stérilisation, placé de préférence verticalement. Les extrémités du tube sont rétrécies et d'une forme telle qu'elles se raccordent aisément à des ligne jetables du commerce, par exemple grâce à des embouts vissables. La cellule 24 est représentée dans sa position stérilisation, c'est à dire que, à la fin d'une dialyse, les lignes ont été déconnectées (et jetées). On voit en 14 les électrodes, en forme de tiges, de plaques ou de bagues en or, en platine, en inox etc., elles sont placées perpendiculairement à la direction de circulation du sang et leurs branchements électriques sont permanents. On voit en 15 schématisée, l'enceinte de confinement qui a la forme d'une boite et qui, à la fin de la dialyse, après que les lignes ont été déconnectées, vient se placer (ou est déjà en place) autour de la cellule de mesure, en appui sur la paroi de la machine de dialyse.

Le système de stérilisation représenté figure 3 est du type à air chaud et sec. Il comporte, intégré à l'enceinte, le dispositif de chauffage qui comprend une résistance chauffante 16 et une cheminée 17. Celle-ci guide l'air ayant pénétré dans l'enceinte par les orifices 18 pour qu'il pénètre dans la cellule. À sa sortie, l'air, représenté par les courants de convection 19 est dirigé vers les orifices 20. Une sonde de contrôle de température 21 est installée au point déterminé comme étant le plus froid. On considère que l'opération de stérilisation est terminée quand en ce point, la température de stérilisation, par exemple 180° C a été atteinte pendant le temps nécessaire.

Le système qui vient d'être décrit avec de la chaleur sèche à la pression atmosphérique peut également être adapté à la chaleur humide, même à des pressions supérieures et l'enceinte 15 se transforme alors en un autoclave.

De même la méthode de stérilisation qu'on vient de décrire pour une cellule de mesure d'impédance sur une machine d'hémodialyse convient pour toute autre technique, même sur d'autres fluides que le sang comme par exemple lors d'une plasmaphérèse, ou bien, sur le sang de nouveau, pour une mesure colorimétrique, de température, de pH... En un mot, chaque fois que les instruments de mesure sont délicats ou chers et ne peuvent être jetés après chaque utilisation ou lorsque leur mise en oeuvre avec des matériels jetables est difficile.

Au lieu des techniques de stérilisation à la chaleur on ne sort pas du cadre de l'invention si l'on utilise d'autres méthodes de stérilisation comme par exemple avec des fluides antiseptiques. Alors le confinement est fait dans une enceinte qui constitue le récipient où est contenu le fluide et qui comporte les tubulures nécessaires pour assurer son arrivée et son départ. L'installation comporte également, dans le cas d'un liquide, un dispositif assurant le séchage avant retrait du récipient-enceinte de confinement.

De même, l'utilisation de rayonnements avec confinement grâce à un moyen permettant l'irradiation de toutes les surfaces à traiter reste dans le domaine de l'invention. C'est à dire que dans ce cas le confinement est obtenu par une irradiation pluridirectionnelle. Celle-ci peut être réalisée dans le cas de la cellule de mesure de l'impédance de la figure 3 en plaçant par exemple une source de rayonnement à chaque extrémité de la cellule en 22, 23. Ici, la garantie d'un fonctionnement correct de la stérilisation est avantageusement obtenue grâce à un détecteur de rayonnement placé à l'emplacement le moins bien exposé aux radiations stérilisantes.

L'invention qu'on vient de décrire permet donc d'effectuer toutes sortes de mesures sur une installation où circulent hors du corps des fluides corporels comme une installation d'hémodialyse, même avec des appareils chers ou qui seraient difficiles à mettre en oeuvre avec des matériels jetables. Elle permet également d'avoir toutes garanties que le fonctionnement a été satisfaisant.

## Revendications

1. Dispositif de mesure et/ou de contrôle placé sur un circuit extra-corporel de fluides corporels, **caractérisé en ce qu'**il comporte des moyens de confinement (15) et de stérilisation (16, 17, 18, 20).

2. Dispositif de mesure et/ou de contrôle selon la revendication 1, **caractérisé en ce que** le moyen de stérilisation est la chaleur et en ce que le confinement est réalisé dans une enceinte (15).

3. Dispositif de mesure et/ou de contrôle selon la revendication 1 ou la revendication 2, **caractérisé en ce que** la source de chaleur est une résistance chauffante (16) placée de préférence en partie basse.

4. Dispositif de mesure et/ou de contrôle selon la revendication 2 et la revendication 3, **caractérisé en ce que** le confinement est réalisé par des courants de convection (19) dans l'enceinte.

5. Dispositif de mesure et/ou de contrôle selon la revendication 2, **caractérisé en ce que** la chaleur est celle d'un courant d'air chaud traversant l'enceinte.

6. Dispositif de mesure et/ou de contrôle selon l'une des revendications 2 à 5, **caractérisé en ce qu'**un détecteur de température (21) est placé à proximité du dispositif et **en ce qu'**on l'utilise comme moyen de contrôle de la stérilisation.

7. Dispositif de mesure et/ou de contrôle selon la revendication 1, **caractérisé en ce que** le confinement est réalisé dans un récipient étanche et **en ce que** le moyen de stérilisation est un liquide désinfectant.

8. Dispositif de mesure et/ou de contrôle selon la revendication 1, **caractérisé en ce qu'**il s'agit d'une cellule de mesure installée sur une machine d'hémodialyse, notamment pour la mesure de l'impédance du sang **et en ce qu'**elle comporte un canal (24) où circule le sang.

9. Dispositif de mesure et/ou de contrôle selon la revendication 8 et l'une des revendications 3 à 5, **caractérisé en ce que** le canal où circule le sang (24) est placé sensiblement verticalement.

10. Dispositif de mesure et/ou de contrôle selon la revendication 1, **caractérisé en ce que** l'agent de stérilisation est un rayonnement et **en ce que** le confinement est obtenu par un rayonnement pluridirectionnel.
